# EUROPÄISCHE PATENTSCHRIFT

(11) **EP 2 803 382 B1**
(45) Veröffentlichungstag und Bekanntmachung des Hinweises auf die Patenterteilung: **21.06.2017**
(21) Anmeldenummer: 14158006.8
(22) Anmeldetag: 06.03.2014
(51) Int. Cl.: A61N 1/362, A61N 1/39

(54) **Externer Herzschrittmacher**
External pacemaker
Stimulateur cardiaque externe

(30) Priorität: 15.05.2013 DE 102013008314
(43) Veröffentlichungstag der Anmeldung: 19.11.2014
(73) Patentinhaber: Osypka AG, 79618 Rheinfelden-Herten (DE)
(72) Erfinder: Bolz, Armin, 91054 Buckenhof (DE); Grämmer, Norbert, 90449 Nürnberg (DE); Seufert, Manuel, 91054 Erlangen (DE); Tröger, Tobias, 91052 Erlangen (DE); Braun, Michael, 89231 Neu-Ulm (DE); Göttsche, Thorsten, 79618 Rheinfelden-Herten (DE)
(74) Vertreter: Herrmann, Uwe

(56) Entgegenhaltungen:
- DE-A1-102011 055 380
- US-A- 5 637 417
- US-A1- 2008 215 119

## Beschreibung

Die vorliegende Beschreibung betrifft einen externen Herzschrittmacher. Die Erfindung ist in den Ansprüche definiert. Herzschrittmacher sind aus dem Stand der Technik bekannt und dienen dazu, den Herzschlag des Nutzers des Herzschrittmachers mittels elektrischer Impulse zu beeinflussen. Hierbei sind sowohl interne Herzschrittmacher, die dem Nutzer komplett implantiert werden als auch externe Herzschrittmacher bekannt, welche vom Nutzer außerhalb seines Körpers getragen werden.

Bei externen Herzschrittmachern ist es dabei bekannt, dass diese über Drehknöpfe und/oder Tasten steuerbar sind. Die Auswahl oder Einstellung eines oder mehrerer Parameter (wie z. B. Stimulationsrate, Stimulationsamplitude, etc.) oder die Auswahl eines Stimulationsmodus wird hierdurch ermöglicht.

Diese Art der Steuerung ist für den Benutzer jedoch meist umständlich und die Parametereinstellung kann unter Umständen längere Zeit in Anspruch nehmen. Weiterhin sind die Drehknöpfe und Tasten aus Platzgründen in geringen Abständen zueinander angebracht, sodass sie schwer bedienbar sind und die Handhabung somit erschwert ist. Auch kann sich zwischen den Drehknöpfen und der Gehäuseschale Schmutz ansammeln, weshalb die Gehäuseschale unterhalb der Drehknöpfe häufig gereinigt werden muss.

Weiterhin ist es bekannt, dass es bei der Handhabung der bekannten Herzschriftmacher häufig zu unbeabsichtigtem Entriegeln bzw. Öffnen des Batteriefachs kommen kann, wobei es geläufig ist, die Ver-/Entriegelungstaste zumindest rudimentär zu sichern. Diese Sicherung ist aber nicht ausreichend und verhindert unbeabsichtigtes Entriegeln nicht wirksam.

Aufgabe der vorliegenden Beschreibung ist es daher, einen externen Herzschrittmacher vorteilhaft weiterzubilden, insbesondere dazu, um die Handhabung des Herzschrittmachers durch die Bedienperson zu verbessern.

Diese Aufgabe wird durch einen Herzschrittmacher mit genau einem Drehknopf zur Steuerung des Herzschrittmachers gelöst. Die Verwendung von genau einem Drehknopf ermöglicht eine einfachere Bedienung und verringert die Komplexität des Herzschrittmachers.

Der Herzschrittmacher gemäß der vorliegenden Beschreibung kann als solcher ausgeführt sein oder mit einem Kardioversionsgerät bzw. einem Defibrillator ausgeführt bzw. verbunden sein.

In einer weiteren Ausführungsform ist wenigstens eine Batteriefachklappe vorgesehen, wobei die Batteriefachklappe mittels einer von einem Schieber verdeckbaren Verriegelung gesichert ist.

Diese Ausführungsform der Erfindung kann alternativ oder zusätzlich zu der Ausführung des Herzschrittmachers verwendet werden, die das Vorhandensein von genau einem Drehknopf vorsieht.

Der Drehknopf kann durch ein mechanisches Bauteil gebildet werden oder auch durch eine Fläche auf einem Touchscreen, die der Nutzer in einer Drehbewegung berühren muss.

Die Betätigung des ausgewählten Menüs kann beispielsweise durch Drücken auf den Touchscreen erfolgen.

Die Steuerung des externen Herzschrittmachers, also z. B. die Auswahl und Einstellung von Stimulationsmodi, Stimulationsparametern, Geräteeinstellungen und Ähnlichem erfolgt überwiegend oder ausschließlich über besagten Drehknopf.

Es sind jedoch weiterhin zusätzliche Tasten möglich, wie z. B. Ein-/Ausschalter, Tasten mit Pausenfunktion, Tasten mit Keylock Funktion, Tasten zum Einstellen der Kardioversion, etc.

Die Stimulationsparameter, Stimulationsmodi und Menüpunkte können dabei auf einem Display des Herzschrittmachers nebeneinander und/oder untereinander angeordnet sein.

Ein aktuell mittels des Drehknopfs ausgewählter Parameter/Modus/Menüpunkt ist beispielsweise durch einen Cursor markiert.

Vorteilhafterweise ist es somit möglich, auf eine Vielzahl von Drehknöpfen zu verzichten und so die Bedienung und die gegebenenfalls nötige Reinigung des Herzschrittmachers zu vereinfachen.

In einem besonders bevorzugten Ausführungsbeispiel ist es denkbar, dass der wenigstens eine Drehknopf zur Steuerung des Herzschrittmachers durch Drehen und/oder durch Drücken betätigbar ist.

Dabei wird beispielsweise der Cursor zur Auswahl eines gewünschten Parameters/Modus/Menüpunkts durch Drehen des Drehknopfs nach rechts/links oder nach unten/oben bewegt. Eine Änderung des ausgewählten Stimulationsparamters kann z. B. durch Drücken des Drehknopfs erfolgen. Hierdurch wird die durch Drehen des Drehknopfs getätigte Auswahl bestätigt.

Ein durch Drücken ausgewählter Parameter kann z. B. dunkel oder farbig auf dem Display hinterlegt werden, um so für den Nutzer besser erkennbar zu sein.

Änderungen der Parameter lassen sich ebenfalls durch Drehen des Drehknopfs und erneutes Drücken des Drehknopfs durchführen und bestätigen. Die genannte Funktionalität des Drehknopfs betrifft gleichfalls die Auswahl, Bestätigung und Änderung von Stimulationsmodi und Menüpunkten.

In einem besonders bevorzugten Ausführungsbeispiel ist denkbar, dass genau ein Drehknopf vorgesehen ist. Ein solches Ausführungsbeispiel mit genau einem Drehknopf stellt sicher, dass die denkbar einfachste Bediengeometrie des Herzschrittmachers bereitstellbar ist.

In einem weiteren bevorzugten Ausführungsbeispiel ist es denkbar, dass der Schieber, der die Verriegelung der Batteriefachklappe abdeckt, durch eine Feder in einer Ausgangsposition positionierbar ist, in der der Schieber die Verriegelung der Batteriefachklappe verdeckt.

Eine so durch den Schieber verdeckte Verriegelung der Batteriefachklappe ist somit gegen unbeabsichtigtes Öffnen der Batteriefachklappe vorteilhaft gesichert. Es kann nicht mehr zu einem ungewollten Öffnen bzw. Entfernen der Batterie aus dem Herzschrittmacher kommen.

An dieser Stelle wird darauf hingewiesen, dass der Begriff "Batterie" allgemein zu verstehen ist und herkömmliche Batterien sowie auch Akkus und sonstige Energiequellen umfasst.

Sollte es zu einem unbeabsichtigten Bewegen des Schiebers und damit zum Freilegen der Verriegelung durch beispielsweise den Anwender kommen, wird der Schieber durch die Federkraft sofort wieder in die Ausgangsposition zurückgeführt. In einem weiteren bevorzugten Ausführungsbeispiel ist es denkbar, dass der Schieber zum Freilegen der Verriegelung gegen die Federkraft der Feder bewegbar ist.

Ein unbeabsichtigtes Bewegen des Schiebers ist daher vorteilhafterweise unwahrscheinlich bzw. erschwert, da der Anwender dabei gegen die Federkraft arbeiten muss. Die Ausführungsbeispiele, die nicht unter den Schutzumfang der beigefügten Ansprüche fallen, dienen lediglich zu illustrativen Zwecken und sind nicht Gegenstand der gegenwärtigen Erfindung. Die vorliegende Breschreibung wird nun anhand von Ausführungsbeispielen und Zeichnungen näher dargestellt. Dabei zeigen:
- Figur 1:: einen Herzschrittmacher mit Schieber und Verriegelung;
- Figur 2a:: einen Drehknopf und eine Bedienoberfläche des Herzschrittmachers;
- Figur 2b:: einen Herzschrittmacher mit Schieber in Ausgangsposition; und
- Figur 2c:: einen Herzschrittmacher mit freigelegter Verriegelung.

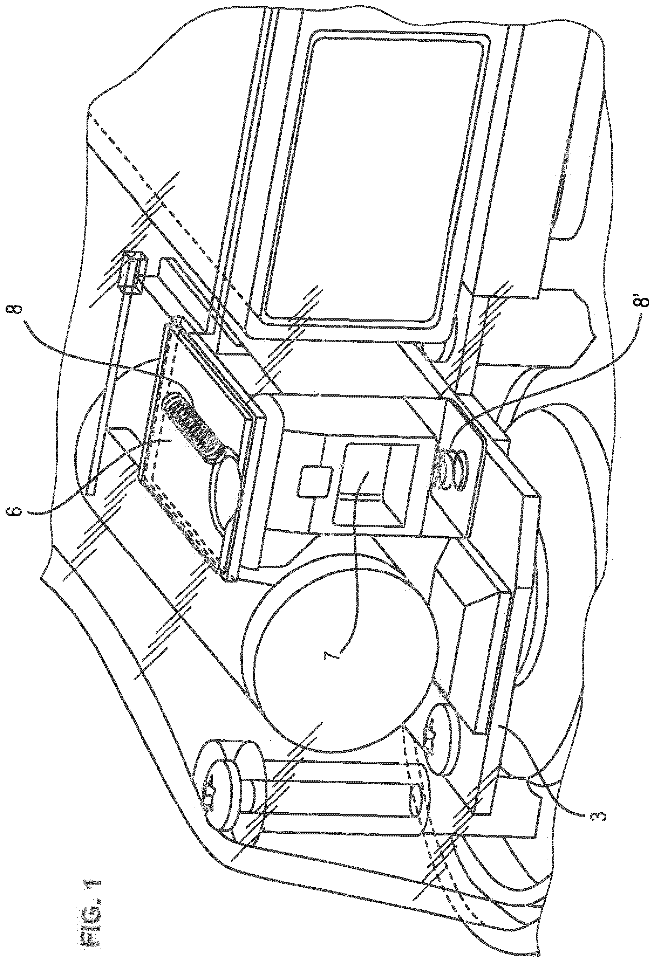

Aus dem Stand der Technik sind externe Herzschrittmacher mit einer Mehrzahl von Drehknöpfen bekannt, wobei mittels der Drehknöpfe unterschiedliche Funktionen bzw. Parameter des Herzschrittmachers eingestellt werden können. Dabei sind ebenfalls Batteriefachklappen bekannt, die mit einfachen bzw. ungesicherten Verriegelungen versehen sein können, mittels derer die Batteriefachklappen ver- und entriegelbar sind. US-A-5,637,417 offenbart einen externen Herzschrittmacher mit einer Batteriefachklappe, die mittels einer Verriegelung gesichert ist. Figur 1 zeigt einen Herzschrittmacher 1 mit Schieber 6 und Verriegelung 7, wobei der Schieber 6 über eine Feder 8 und die Verriegelung 7 über eine Feder 8' positionierbar bzw. rückstellbar ist. Im unteren Bereich des Herzschrittmachers 1 ist die Batteriefachklappe 3 zu erkennen, die mittels der Verriegelung 7 ver- und entriegelbar ist.

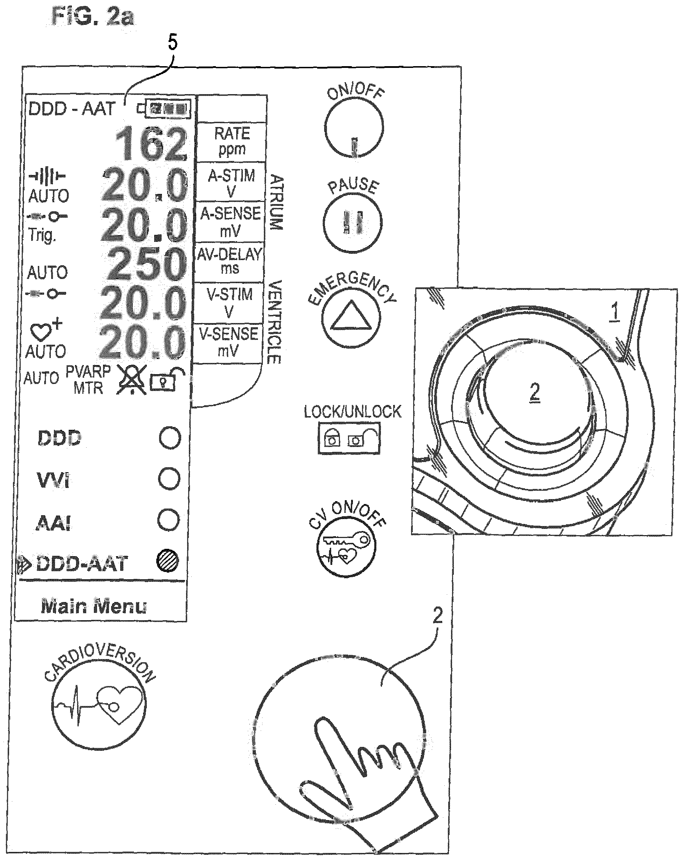

Figur 2a zeigt rechts einen Drehknopf 2 an einem Gehäuseabschnitt des Herzschrittmachers 1 sowie links eine schematische Darstellung des Displays 5 eines Herzschrittmachers 1, verschiedenen Tasten und den Drehknopf 2. Angedeutet ist dabei, dass durch Drehen des Drehknopfs 2 die vertikal angeordneten Einstellungsbereiche angesteuert und geändert werden können.

Denkbar ist es, dass durch Drehen des Drehknopfes 2 eine Auswahl aus einem Menü erfolgt und dass die Bestätigung der ausgewählten Menüfunktion durch Drücken dieses Drehknopfes 2 oder auch eines anderen Betätigungselementes erfolgt.

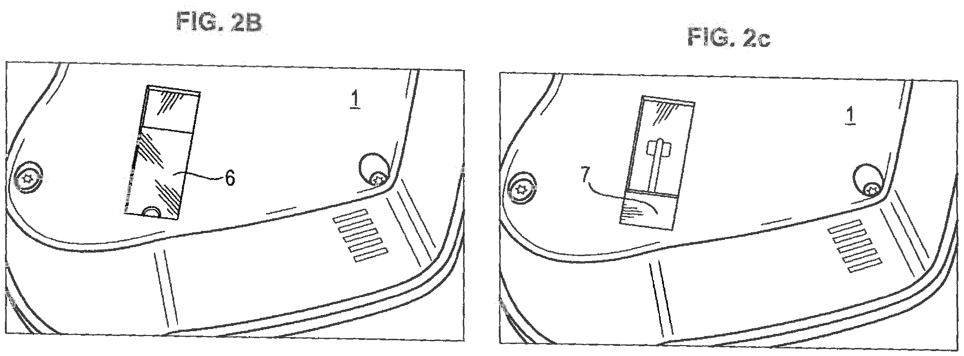

Figur 2b zeigt einen Abschnitt des Herzschrittmachers 1, an dem der Schieber 6 vorgesehen ist, mittels welchem die Verriegelung 7 abdeckbar und dadurch sicherbar ist und welcher in Figur 2c ausgeblendet ist.

In Figur 2c ist die unter dem Schieber 6 vorgesehen Verriegelung 7 erkennbar, mittels derer die hier nicht gezeigte Batteriefachklappe 3 ver- und entriegelbar ist.

Das Entriegeln des Batteriefachs erfolgt durch Betätigung der Verriegelung 7, indem der Nutzer mit dem Finger die Verriegelung 7 in den Herzschrittmacher eindrückt. Hierzu muss er den Schieber 6 zunächst gegen die Federkraft der Feder 8 bewegen. Erst dann ist die Verriegelung 7 zugänglich.

Befindet sich der Schieber 6 in seiner nicht bewegten Ausgangsstellung, ist die Verriegelung 7 nicht zugänglich und somit das Batteriefach gegen Öffnen geschützt. Die Erfindung wird durch die folgenden Ansprüche definiert:

## Patentansprüche

1. Externer Herzschrittmacher (1) mit wenigstens einer Batteriefachklappe (3), **dadurch gekennzeichnet, dass** die Batteriefachklappe (3) mittels einer von einem Schieber (6) verdeckbaren Verriegelung (7) gegen unbeabsichtigtes Öffnen der Batteriefachklappe gesichert ist.

2. Externer Herzschrittmacher (1) nach Anspruch 1, **dadurch gekennzeichnet, dass** er genau einem Drehknopf (2) zur Steuerung des Herzschrittmachers (1) umfasst.

3. Externer Herzschrittmacher (1) nach Anspruch 2, **dadurch gekennzeichnet, dass** der Drehknopf (2) zur Steuerung des Herzschrittmachers (1) durch Drehen und/oder durch Drücken betätigbar ist.

4. Externer Herzschrittmacher (1) nach Anspruch 1, **dadurch gekennzeichnet, dass** mehrere Drehknöpfe (2) vorgesehen sind.

5. Externer Herzschrittmacher (1) nach einem der Ansprüche 1 bis 4, **dadurch gekennzeichnet, dass** der Schieber (6) durch eine Feder in einer Ausgangsposition positionierbar ist, in der der Schieber (6) die Verriegelung (7) der Batteriefachklappe (3) verdeckt.

6. Externer Herzschrittmacher (1) nach einem der Ansprüche 1 bis 5, **dadurch gekennzeichnet, dass** der Schieber (6) zum Freilegen der Verriegelung (7) gegen die Federkraft der Feder bewegbar ist.

7. Externer Herzschrittmacher (1) nach einem der Ansprüche 2, 3 oder 4 bis 6, **dadurch gekennzeichnet, dass** es sich bei dem Drehknopf um einen mechanischen Drehknopf handelt.

8. Externer Herzschrittmacher (1) nach einem der Ansprüche 2, 3 oder 4 bis 6, **dadurch gekennzeichnet, dass** es sich bei dem Drehknopf um eine Fläche eines Touchscreen-Bildschirms handelt.

## Claims

1. An external pacemaker (1) having at least one battery compartment cover (3), **characterized in that**
the battery compartment cover (3) is secured against an unintentional opening of the battery compartment cover by means of a locking mechanism (7) coverable by a slide (6).

2. An external pacemaker (1) in accordance with claim 1, **characterized in that** it comprises exactly one rotary knob (2) for controlling the pacemaker (1).

3. An external pacemaker (1) in accordance with claim 2, **characterized in that** the rotary knob (2) for controlling the pacemaker (1) is actuable by turning and/or pressing.

4. An external pacemaker (1) in accordance with claim 1, **characterized in that** a plurality of rotary knobs (2) are provided.

5. An external pacemaker (1) in accordance with one of the claims 1 to 4, **characterized in that** the slide (6) can be positioned by a spring in a starting position in which the slide (6) covers the locking mechanism (7) of the battery compartment cover (3).

6. An external pacemaker (1) in accordance with one of the claims 1 to 5, **characterized in that** the slide (6) is movable against the spring force of the spring to expose the locking mechanism (7).

7. An external pacemaker (1) in accordance with one of the claims 2, 3 or 4 to 6, **characterized in that** the rotary knob is a mechanical rotary knob.

8. An external pacemaker (1) in accordance with one of the claims 2, 3 or 4 to 6, **characterized in that** the rotary knob is an area of a touchscreen monitor.

## Revendications

1. Stimulateur cardiaque externe (1) comprenant au moins un couvercle de compartiment des piles (3), **caractérisé en ce que**
le couvercle de compartiment des piles (3) est protégé contre une ouverture involontaire du couvercle de compartiment des piles au moyen d'un mécanisme de verrouillage (7) qui peut être caché par un élément coulissant (6).

2. Stimulateur cardiaque externe (1) selon la revendication 1, **caractérisé en ce qu'**il comprend exactement un bouton tournant (2) pour la commande du stimulateur cardiaque (1).

3. Stimulateur cardiaque externe (1) selon la revendication 2, **caractérisé en ce que** le bouton tournant (2) pour la commande du stimulateur cardiaque (1) peut être actionné par rotation et/ou par pression.

4. Stimulateur cardiaque externe (1) selon la revendication 1, **caractérisé en ce que** plusieurs boutons tournants (2) sont prévus.

5. Stimulateur cardiaque externe (1) selon l'une des revendications 1 à 4, **caractérisé en ce que** l'élément coulissant (6) peut être positionné par un ressort dans une position initiale, dans laquelle l'élément coulissant (6) cache le mécanisme de verrouillage (7) du couvercle de compartiment des piles (3).

6. Stimulateur cardiaque externe (1) selon l'une des revendications 1 à 5, **caractérisé en ce que** l'élément coulissant (6) peut être déplacé contre la force de ressort du ressort pour dégager le mécanisme de verrouillage (7).

7. Stimulateur cardiaque externe (1) selon l'une des revendications 2, 3 ou 4 à 6, **caractérisé en ce que** le bouton tournant est un bouton tournant mécanique.

8. Stimulateur cardiaque externe (1) selon l'une des revendications 2, 3 ou 4 à 6, **caractérisé en ce que** le bouton tournant est une surface d'un écran tactile.
